# EUROPEAN PATENT SPECIFICATION

(11) **EP 0 957 788 B1**
(45) Date of publication and mention of the grant of the patent: **27.10.2004**
(21) Application number: 96908946.5
(22) Date of filing: 04.04.1996
(51) Int. Cl.: A61B 17/34, A61C 3/02, A61M 25/06

(54) **TARGETED NEEDLE DRUG ADMINISTRATION**
EINE GEZIELTE VERABREICHUNG EINER ARZNEI MITTELS EINER NADEL
ADMINISTRATION CIBLEE D'UN MEDICAMENT A L'AIDE D'UNE AIGUILLE

(30) Priority: 07.04.1995 CA 2146585
(43) Date of publication of application: 24.11.1999
(73) Proprietor: Gibbs, David E., Ottawa, Ontario K1Y 2X1 (CA)
(72) Inventor: Gibbs, David E., Ottawa, Ontario K1Y 2X1 (CA)
(74) Representative: McKechnie, Neil
(86) International application number: PCT/CA1996/000203
(87) International publication number: WO 1996/031164

(56) References cited:
- EP-A- 0 292 252
- US-A- 4 356 828
- US-A- 4 969 870
- US-A- 5 332 398
- US-A- 5 354 299
- US-A- 5 372 583

## Description

### BACKGROUND OF THE INVENTION

### 1. Field of the invention

The present invention provides for a simple method and device for infusing or injecting medication; it is applicable to medical or dental and the like procedures. More particularly, the invention is directed to catheterized delivery of anaesthesia and other medication. More particularly still, it is directed to catheterized delivery of dental anaesthetic to a targeted nerve and to an apparatus for such delivery.

### 2. Related Art

There are a variety of methods currently in use for providing local anaesthetic in dentistry. These methods and apparatuses however all have disadvantages, either being difficult for practitioners to perform or painful and unpleasant to the patient.

An example of a method used currently in dentistry is the infiltration method, whereby a local anaesthetic solution is injected into the soft tissue of gingiva. In doing so, the solution eventually passes through the cortical plate affecting the nerve bundle entering the tooth. Disadvantages of this method include the delay of onset of anaesthesia after the injection and, in most cases, ballooning of the injected tissue. As well, there is an extended period of time for recovery of the tissue until return to normal condition.
Another method which is currently used is the regional block method whereby an anaesthetic solution is injected locally in proximity to the nerve trunk as it enters the bone. Disadvantages of this procedure are that it is extremely difficult to locate the nerve trunk, there is discomfort to the patient and a delay for the anaesthetic to take effect. As in the case of the infiltration method, this method necessitates a long recovery period for tissue to return to normal.

At present, two types of apparatus have been used to perform intra-osseous anaesthesia. These are surgical burs used to perforate the cortical plate and the villet injectors.

The use of a surgical bur has disadvantages in that burs are expensive and they have to be sterilized between uses or a new bur used each time. In addition, the method is slow, requiring the attached gingiva and periosteum to be anaesthetized before the cortical plate is perforated. The villet injector is an apparatus that serves both as a perforator and injector. It uses specially designed needles rotated by a conventional dental motor. A disadvantage of this device is that the needle often becomes clogged with pulverized bone which obstructs the passage in the needle and prevents injection of the anaesthetic solution. It is generally difficult to remove the clogging material from the needle and often the use of a second needle is necessary. Other disadvantages of this method include the initial capital cost of the instrument purchase, and the cost of the needles which are somewhat expensive. In addition, the design of the instrument makes access to various parts of the mouth difficult and sometimes impossible.

Intra-osseous and targeted root-canal nerve anaesthesia have not become popular for the reason that there has not been a practical technique of making the injections successfully. For example, there has been a general belief that this method is radical and to be restored to only if nerve block and infiltration anaesthetic do not accomplish the desired result. However, intra-osseous and targeted injections produce positive, more profound anaesthesia and could be made with less pain than either of the other types according to the present invention.

Targeted anaesthesia has several advantages over prior art nerve block or infiltration methods. There is no feeling of numbness in the tongue, cheek, or lips during or after the injection and there is no after-pain. The anaesthetic is profound and acts immediately alleviating the necessity of waiting for the anaesthetic to take effect as with the nerve block and infiltration methods. Furthermore, as only a few drops of anaesthetic are injected, there is no feeling of faintness or increasing of the pulse rate.

To achieve targeted anaesthesia one must gain access, if intra-osseous, to the cancellous bone by going through the cortical layer; or to the bottom of the tooth, if root-canal targeted anaesthesia is desired. Because of instant anaesthesia and profound pulpal anaesthesia, there is a much greater control over the region one wishes to anaesthetize, resulting in a much smaller dose of anaesthetic; as well as, of course, other medication, where applicable.

United States Patent 5,173,050 (Dillon) discloses a dental apparatus for perforating the cortical plate of human maxillary and mandibular bones. The apparatus of Dillon comprises a metal needle moulded into a plastic shank. The shank is being formed with means for cooperation with a dental hand piece for transmitting the rotational movement to the needle. The needle used for drilling is solid and has a sharp bevelled free end. The apparatus described by Dillon is disposable.

However, the device disclosed in Dillon's patent cannot be used as a catheter for injecting anaesthetic by inserting a hypodermic needle through the drilling needle. As well, the device disclosed by Dillon is not provided with means for blocking entry of bone debris into the needle passageway. In addition, the direct connection between the hand piece and the perforator does not provide for a safe and reliable barrier against bacteria passing from the needle to the hand piece.

United States Patent 3,534,476 (Winters) discloses a drilling and filling root canal apparatus. The drilling is performed by a drill having a central bore. The depth of the root canal is determined in advance and a stop is placed on the drill to limit the depth of drilling. The device is provided with a flexible rod which is pushed into the root canal so that the drill is directed along this road to follow the contour of the canal so that resulting bore will have an uniform diameter which is free of shoulders or ledges. The apparatus disclosed by Winters is concerned with enlarging the root canal after the nerve has been extracted. This apparatus is not used for injecting medication in close proximity to a targeted area for treatment or anaesthetic.

United States Patent 4,944,677 (Alexandre) discloses a smooth hollow needle with a bevelled point for drilling a hole into the jawbone near the apex of the tooth to be anaesthetized. Thereafter, the drilling device 13 removed from the jaw, and a hypodermic needle of substantially the same gauge is inserted into the hole and anaesthesia is injected. Thus, there is no cathetized delivery of medication, with the attendant disadvantage that the pre-drilled hole may be difficult to locate when inserting the hypodermic needle.

One significantly older United States patent that is discussed by Alexandre (above) is United States Patent 2,317,648 (Siqveland) granted in 1943. In addition to the disadvantage mentioned by Alexandre, the fact that Siqveland teaches use of threaded sleeve which penetrates the bone during drilling and is left (screwed) in the bone to serve as a guide for insertion of the actual injection needle. Due to the cost of such a device, it cannot be made disposable; but more importantly, for the threaded sleeve to be securely fastened in the bone it would have to rotate at a much slower speed than the drill (as in Siqveland) or the drilling catheter (as in the present invention).

US 5332 398 which is the closest prior art, discloses an apparatus for use in the delivery or withdrawal of fluid to or from the vascular system of a patient's bone marrow. The apparatus uses an intermedullary catheter having a head and a threaded conduit device. The apparatus is manually rotated so as to cause the threaded conduit to engage with the bone of the patient. In this document, it is taught that manual rotation of the intermedullary catheter requires a specialist tool.

### SUMMARY OF THE INVENTION

The present invention endeavours to mitigate the problems and disadvantages of delivering dental anaesthetic encountered with the prior art methods and devices.

According to the present invention, there is provided a rotatable drilling device for use in injecting medication or anesthetic, said rotatable drilling device comprising: a rotatable needle hub and a hollow drilling needle, having a first outer diameter, and extending outwardly from an end of the rotatable needle hub, wherein the hollow drilling needle has a sharp tip at a first end, and the rotatable needle hub has a bore extending therethrough through which medication or anesthetic can be introduced into the bore and into the hollow drilling needle
characterised in that
the rotatable drilling device further comprises an adapter having a rod at a lower end and a shank, the rotatable needle hub being adapted for coupling with the adapter, wherein the rod is suitable for removable insertion into the bore of the rotatable needle hub and through into the hollow drilling needle such that when so inserted the rod substantially prevents debris from entering the hollow drilling needle and blocking the hollow needle during drilling, and the shank is adapted to removably couple with a motorised handpiece for rotation therewith when coupled thereto.

The apparatus in accordance with the present invention is further defined in the dependent Claims.

In the preferred form of the invention the apparatus is disposable. Before disposal, the rotatable drilling device receives a cap over the needle for protection against accidental contamination of environment.

Advantageously, the device of the present invention provide users with a more secure and less painful method and device for direct access for injecting medication to a target area into the cortical plate of the bone.

In addition, the device facilitates and adds a level of security previously unavailable for the anaesthetic in that it has a sure and immediate effect.

Another advantage of this device is that it provides benefits to the dentists by facilitating the use of a low cost, disposable device.

Still another advantage of this invention is that the risk of contamination is lower than with the current devices. This is because the device is disposable and because the risk of the dental equipment used with the device of the invention becoming contaminated is low.

### BRIEF DESCRIPTION OF THE DRAWINGS

These and other features of the invention will become more apparent from the following description of the preferred embodiments, in which reference is made to the appended drawings, wherein:
Figure 1 illustrates an exploded view of the device showing the component parts and their inter-relationship;
Figure 2A illustrates the device assembled for drilling;
Figure 2B Illustrated a longitudinal cross-section through the device illustrated in Figure 2A, taken along line A-A of Figure 2A;
Figure 3 shows a detailed view of the area marked on Figure 2B;
Figure 4 is a cross-sectional view of the body of the perforator taken along lines B-B of Figure 2;
Figure 5 is a cross-sectional view of the adaptor body;
Figure 6 is a cross-sectional view of the cap;
Figure 7A - 7C illustrate the method according to the invention, Figure 7A shows the device drilling in the bone tissue; Figure 7B shows the perforator inserted into the bone tissue and the adaptor de-coupled; and Figure 7C shows the perforator inserted into the bone tissue as a catheter and a hypodermic needle set for delivering an injection;
Figure 8 illustrates another embodiment of the invention;
Figure 9 illustrates an alternative method of delivery medication to treat a root-canal nerve; and
Figure 10 illustrates from a plan view the point of catheter insertion for the alternative method shown in Figure 9.

### DETAILED DESCRIPTION OF THE PREFERRED EMBODIMENT

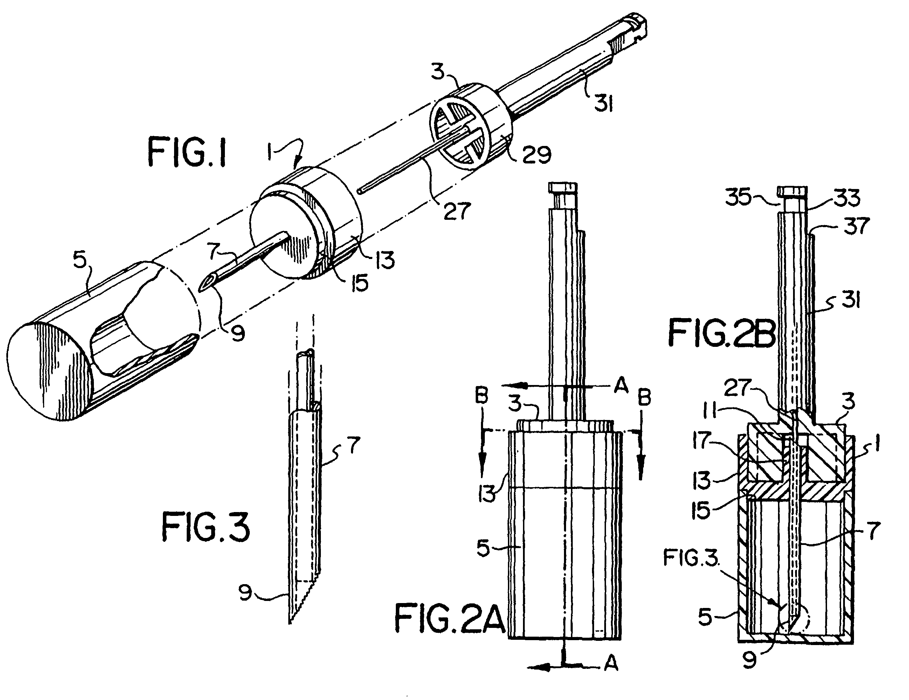

Figure 1 illustrates an exploded view of the device showing the component parts and their inter-relationship. The device comprises a perforator 1, an adaptor 3 and a cap 5.

The perforator 1 has a bevelled drilling needle 7 which is used both as a drill and a catheter. Needle 7 is bevelled at both ends, as better shown on Figure 2B. The first end 9 is formed as a drilling tip in that it has cutting teeth along the edge, as shown in Figure 3. The second end 11 is bevelled for receiving and directing the needle of a hypodermic syringe and for easy coupling with the adaptor 3, as will be seen later.

A flange 13 is fixed on the needle about the second end 11, so that the needle passes along the geometrical axis of the flange 13. The flange 13 is manufactured or moulded of a plastic or other material, and it has a generally cylindrical outer shape. This shape is preferred as the flange 13 rotates together with the needle 7 for drilling. Variations of the shape illustrated in the attached drawings may also be contemplated.

The flange 13 is adapted for receiving cap 5 at one end and for coupling with the adaptor 3 at the other end. As an example, a collar 15 may be provided on the flange 13 so that the cap 5 holds over the collar 15 when pressed. The cap 5 is needed to protect and cover the tip 9 of the needle 7 before use and when the device is disposed of.

For ease of manipulation the internal diameter of the cap 5 and the external diameter of the collar 15 should be as large as is reasonable and preferably between 10 to 20 times greater than the diameter of the needle 7. Another advantage of the collar 15 is that it provides a stop to limit the depths of penetration of the needle 7. The flange 13 is shaped to form an inner axial shaft 17 projecting from the centre of the collar 15, and a female connector 19 for coupling with a corresponding male connector provided in the adaptor 3.

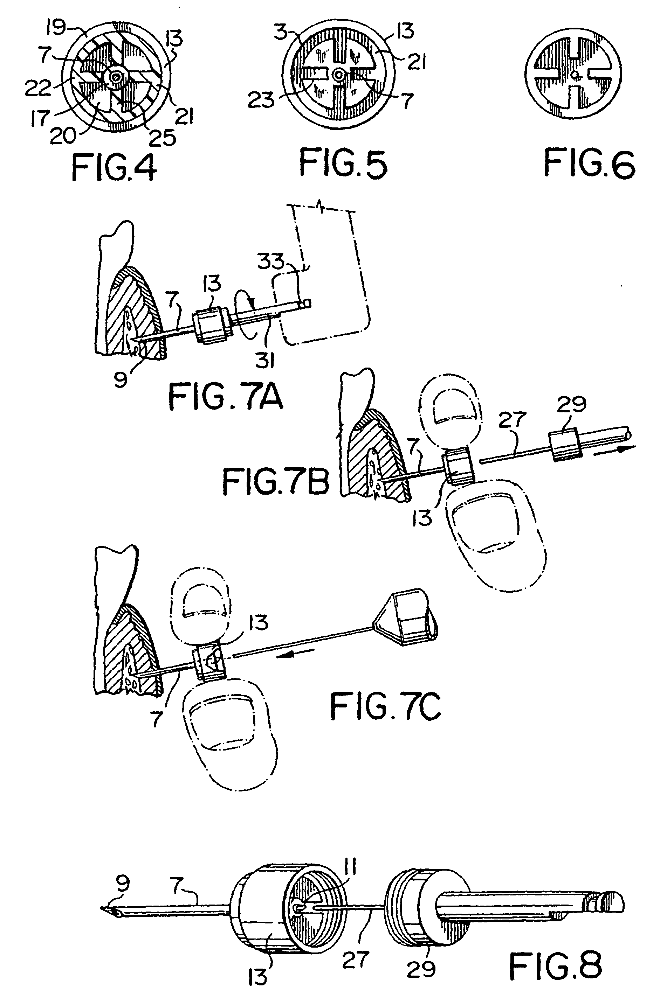

The coupling between the perforator and the adaptor is illustrated on Figures 2B, 4 and 5. Figure 2B shows a longitudinal section of a female connector 19 provided in the flange 13 and a male connector 21 provided in the adaptor 3. Figure 4 illustrates a cross-section of an exemplary female connector 19, while Figure 5 shows a cross-section of the corresponding male connector 21. The male connector is provided with radial ribs 23, extending towards the centre but not meeting to leave room for the central shaft 17, while corresponding grooves 25 are provided in the female connector, alternating with islands 20. The female connector is also formed with a clearance ring 22 for accommodating the thickness of the body 29 of the adaptor.

The tubular shaft 17 forms a reinforced passage for drilling needle 7. The shaft also provides enough contact surface between the drilling needle and the body to ensure that these two parts rotate together during drilling. As could be seen on Figure 2B, end 11 of the needle is bevelled and extends a little over the shaft 17, but there is a clearance between the tip of end 11 and the male connector when the device is assembled for drilling.

When rotated, the drilling needle 7 penetrates in the bone tissue through gingiva or ligament and drills a hole with the cutting tip 9. The perforator 1 may remain in place as a catheter, with the drilling needle inserted into the bone. Then, a hypodermic needle may be introduced through the passage of drilling needle 7 to inject a medicament directly into the bone. Therefore, the drilling needle 7 is selected to have a wide enough passage for allowing a hypodermic needle with a smaller gauge to be inserted through needle 7.

The adaptor 3 has three functions. Firstly, the adaptor conveys the rotational movement from a dental hand piece or the like to the perforator. As well, the adaptor is provided with means for blocking bone debris for entering into the syringe passage and also aligns and reinforces the needle 7 during drilling.

The adaptor includes a rod 27, a body 29 and a shank 31.

Body 29 includes male connector 21 which is formed, as indicated above, with longitudinal ribs 23 which couple with grooves 25 of the female connector 19 for driving needle 7. The shank 31 extends along the axis of the adaptor and is formed with a joint 33 for attachment with a contra-angle or straight hand piece. The shank 31 has a groove 35 and a cut-out 37 to fix the shank in place in the known manner. Generally, the shank transmits to the needle 7 the rotational movement from the hand piece.

The shank 31 also acts as a barrier for contamination, at it is generally thought that bacteria is reluctant to change direction, and there are a plurality of 90° angles between the tip 9 of drilling needle 7 and the joint 33.

The rod 27 has the diameter and length selected in accordance with the size of needle 7. The rod 27 is fixed in the geometrical centre of body 29 so as to readily penetrate into the hollow passage of the needle, when the device is assembled for drilling. When the rod 27 is inserted within the needle passage, it advances through the length of the needle up to the bevelled end, as shown on Figures 2A and 3 in dotted lines. In this way, the debris from drilling cannot penetrate to block the needle passage. In addition, the rod gives additional rigidity, strength and alignment to needle 7 during drilling. The rod also advances through the a portion of the shank as is illustrated in Figure 2B in dotted lines.

Figure 8 illustrates an alternative embodiment of the present invention. In this variant, body 13 is provided with an internal thread while body 29 is provided with a matching external thread. By threading one to the other and using the central rod 27 to align the two bodies together, the perforator could be driven by the hand piece in a similar manner as in the variant disclosed above. Of course, the thread is going in an opposite direction to the direction of rotation of the device for avoiding disconnection of the two bodies.

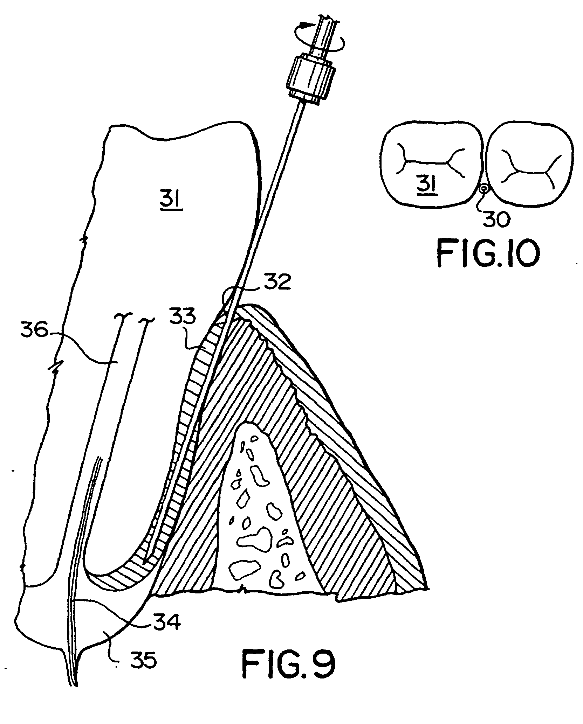

An alternative method of targeted delivery is shown in Figures 9 and 10. The perforator 7 is inserted at a point 30 between teeth, parallel to the tooth 31 in treatment, and penetrates through gingival sulcus 32 and ligament 33 to a depth near the entry of the nerve, artery and vein bundle 34 through the bone 35 and into the tooth-root canal 36. This method of targeted delivery, say, of anaesthesia is suitable, where perforating vertical to the tooth through gingiva and cortical bone is not convenient or possible; as in the case of rear molars.

There are a variety of ways that this invention can be devised but the end result is to perform catheterized intra-osseous delivery system.

The device of this invention operates as follows:

First, a site for the injection is selected by the practitioner. The gingiva over the injection side is disinfected and topically anaesthetized. A small amount of anaesthetic solution is injected until blanching of the tissue, and this will anaesthetize the gingiva and the periosteum. The following operations are illustrated in Figures 7A, 7B and 7C, and Figures 9 and 10.

As can be seen in Figure 7A, the bevelled end 9 of the needle 7 is placed against the gingiva and shank 31 is attached with joint 33 to a contra angle or to a straight dental hand piece. The adaptor and perforator are coupled for drilling. The perforator should be held perpendicular to the cortical plate, or if not possible or convenient, it should be held vertical and parallel to the long axis of the tooth as shown in Figure 9, having been inserted between teeth as shown in Figure 10. The perforator is then operated in small bursts of rotation from the hand piece until resistance is no longer felt, as is well known to dentists.

Next, the adaptor 3 is removed from the engagement with perforator 1 by applying pressure to the body 13 with the fingers thus keeping the needle 7 in the perforated cortical plate. This is shown in Figure 7B.

The presence of the needle 7 in the cortical plate, or down the side of the tooth as in Figure 9, allows an injection to be made without complicated manoeuvres to find the perforation in the case of floating gingiva or the free or marginal gingiva. Figure 7C illustrates the next step, namely how the injection needle is inserted through the perforator 1 for delivering the anaesthetic solution required.

The last step is to remove the perforator 1 from the cortical plate and reinstall the cover cap 5 over the needle 7, then insert the adaptor to the perforator making the unit complete and disposable. The cap 5 provides a means whereby the apparatus may be removed from the dental hand piece without any risk of the user being in contact with body fluids which will be present on the needle after use. This is extremely important particularly since there may be a risk of contacting Aids or Hepatitis should the user accidentally prick a finger with the needle. It is therefore desirable that the cap should be of a hard or rigid rubber or plastic material not easily penetrated by the needle.

## Claims

1. A rotatable drilling device for use in injecting medication or anesthetic, said rotatable drilling device comprising:
a rotatable needle hub (13) and a hollow drilling needle (7), having a first outer diameter, and extending outwardly from an end of the rotatable needle hub (13)
wherein the hollow drilling needle (7) has a sharp tip (9) at a first end, and the rotatable needle hub (13) has a bore (17) extending therethrough through which medication or anesthetic can be introduced into the bore (17) and into the hollow drilling needle (7)
**characterised in that** the rotatable drilling device further comprises an adapter (3) having a rod (27) at a lower end and a shank (31), the rotatable needle hub (13) being adapted for coupling with the adapter (3)
wherein the rod (27) is suitable for removable insertion into the bore (17) of the rotatable needle hub (13) and through into the hollow drilling needle (7) such that when so inserted the rod (3) substantially prevents debris from entering the hollow drilling needle (7) and blocking the hollow drilling needle (7) during drilling,
and the shank (31) is adapted to removably couple with a motorised handpiece for rotation therewith when coupled thereto.

2. A rotatable drilling device as defined in Claim 1, wherein the sharp tip (9) is suitable for drilling a hole into ligament, bone or tissue.

3. A rotatable drilling device as defined in Claims 1 or Claim 2, wherein the diameter of the hollow drilling needle (7) is sized to receive a hypodermic needle for delivery of medication.

4. A rotatable drilling device as defined in Claims 1 to 3, wherein the sharp tip (9) comprises a bevelled cutting edge.

5. A rotatable drilling device as defined in Claims 1 to 4, wherein the hollow drilling needle (7) further comprises a bevelled catheter tip (11) at a second end.

6. A rotatable drilling device as defined in Claim 1 to 5 wherein the rod (27) has a smaller diameter than the hollow drilling needle (7).

7. A rotatable drilling device as defined in Claims 1 to 6 wherein the rod (27) has an outer diameter that is substantially the same as an inner diameter of the hollow drilling needle (7).

8. A rotatable drilling device as defined in Claims 1 to 7, wherein the adapter further comprises a complementary adapter hub (29) for mating with the rotatable needle hub (13) and for locking therewith, such that during locking engagement rotation of the adapter hub (29) rotates the rotatable needle hub (13).

9. A rotatable drilling device as defined in Claims 1 to 8, wherein the device further comprises a cap (5) for mating with the rotatable needle hub (13) such that when mated with the rotatable needle hub (13) the cap (5) isolates and protects the sharp tip (9) and the hollow drilling needle (7).

## Patentansprüche

1. Rotierfähige Bohrvorrichtung zur Verwendung beim Injizieren von Arzneimitteln oder Anästhetika, wobei die rotierfähige Bohrvorrichtung Folgendes umfasst:
eine rotierfähige Nadelnabe (13) und eine hohle Bohrnadel (7), die einen ersten Außendurchmesser hat und sich von einem Ende der rotierfähigen Nadelnabe (13) nach außen erstreckt,
wobei die hohle Bohrnadel (7) an einem ersten Ende eine scharfe Spitze (9) hat und die rotierfähige Nadelnabe (13) eine durch sie hindurch verlaufende Bohrung (17) hat, durch die ein Arzneimittel oder Anästhetikum in die Bohrung (17) und in die hohle Bohrnadel (7) eingeführt werden kann,
**dadurch gekennzeichnet, dass** die rotierfähige Bohrvorrichtung ferner einen Adapter (3) mit einer Stange (27) an einem unteren Ende und einem Schaft (31) umfasst, wobei die rotierfähige Nadelnabe (13) zum Verbinden mit dem Adapter (3) ausgebildet ist,
wobei die Stange (2) zum entfernbaren Einführen in die Bohrung (17) der rotierfähigen Nadelnabe (13) und hindurch in die hohle Bohrnadel (7) geeignet ist, sodass die so eingeführte Stange (3) im Wesentlichen verhindert, dass Schmutz in die hohle Bohrnadel (7) eindringt und die hohle Bohrnadel (7) während des Bohrens verstopft,
und der Schaft (31) ausgebildet ist, um entfernbar mit einem kraftbetriebenen Handgerät verbunden zu werden zur Drehung damit, wenn er damit verbunden ist.

2. Rotierfähige Bohrvorrichtung nach Anspruch 1, bei der die scharfe Spitze (9) zum Bohren eines Loches in Band, Knochen oder Gewebe geeignet ist.

3. Rotierfähige Bohrvorrichtung nach Anspruch 1 oder Anspruch 2, bei der der Durchmesser der hohlen Bohrnadel (7) zum Aufnehmen einer Subkutannadel zur Abgabe von Arzneimittel bemessen ist.

4. Rotierfähige Bohrvorrichtung nach einem der Ansprüche 1 bis 3, bei der die scharfe Spitze (9) eine abgeschrägte Schneide hat.

5. Rotierfähige Bohrvorrichtung nach einem der Ansprüche 1 bis 4, bei der die hohle Bohrnadel (7) ferner an einem zweiten Ende eine abgeschrägte Katheterspitze (11) hat.

6. Rotierfähige Bohrvorrichtung nach einem der Ansprüche 1 bis 5, bei der die Stange (27) einen kleineren Durchmesser als die hohle Bohrnadel (7) hat.

7. Rotierfähige Bohrvorrichtung nach einem der Ansprüche 1 bis 6, bei der die Stange (27) einen Außendurchmesser hat, der im Wesentlichen der gleiche wie ein Innendurchmesser der hohlen Bohrnadel (7) ist.

8. Rotierfähige Bohrvorrichtung nach einem der Ansprüche 1 bis 7, bei der der Adapter ferner eine komplementäre Adapternabe (29) zum Zusammenpassen mit der rotierfähigen Nadelnabe (13) und zum Sichern daran umfasst, sodass während des Sicherungseingriffs die Drehung der Adapternabe (29) die rotierfähige Nadelnabe (13) dreht.

9. Rotierfähige Bohrvorrichtung nach einem der Ansprüche 1 bis 8, bei der die Vorrichtung ferner eine Kappe (5) zum Zusammenpassen mit der rotierfähigen Nadelnabe (13) umfasst, sodass die Kappe (5), wenn sie mit der rotierfähigen Nadelnabe (13) zusammengepasst ist, die scharfe Spitze (9) und die hohle Bohrnadel (7) isoliert und schützt.

## Revendications

1. Dispositif de forage rotatif à utiliser pour injecter un médicament ou un anesthétique, ledit dispositif de forage rotatif comprenant :
un moyeu d'aiguille rotatif (13) et une aiguille de forage creuse (7), ayant un premier diamètre externe, et s'étendant vers l'extérieur depuis une extrémité du moyeu d'aiguille rotatif (13) ;
dans lequel l'aiguille de forage creuse (7) comporte une pointe acérée (9) à une première extrémité, et le moyeu d'aiguille rotatif (13) comporte un alésage (17) s'étendant à travers celui-ci par lequel un médicament ou un anesthétique peut être introduit dans l'alésage (17) et dans l'aiguille de forage creuse (7) ;
**caractérisé en ce que** le dispositif de forage rotatif comprend en outre un adaptateur (3) comportant une tige (27) à une extrémité inférieure et une queue (31), le moyeu d'aiguille rotatif (13) étant à même d'être accouplé à l'adaptateur (3) ;
dans lequel la tige (27) peut être insérée de manière amovible dans l'alésage (17) du moyeu d'aiguille rotatif (13) et à travers celui-ci dans l'aiguille de forage creuse (7) de sorte que, lorsqu'elle est introduite de cette manière, la tige (3) empêche en grande partie que des débris ne pénètrent dans l'aiguille de forage creuse (7) et ne bloquent l'aiguille de forage creuse (7) pendant le forage, et
la queue (31) est à même d'être accouplée de manière amovible à une pièce à main motorisée pour tourner avec celle-ci lorsqu'elle est accouplée à celle-ci.

2. Dispositif de forage rotatif suivant la revendication 1, dans lequel la pointe acérée (9) est appropriée pour forer un trou dans un ligament, un os ou un tissu.

3. Dispositif de forage rotatif suivant la revendication 1 ou 2, dans lequel le diamètre de l'aiguille de forage creuse (7) est dimensionné pour recevoir une aiguille hypodermique pour l'administration d'un médicament.

4. Dispositif de forage rotatif suivant l'une quelconque des revendications 1 à 3, dans lequel la pointe acérée (9) comprend une arête tranchante en biseau.

5. Dispositif de forage rotatif suivant l'une quelconque des revendications 1 à 4, dans lequel l'aiguille de forage creuse (7) comprend en outre une pointe de cathéter en biseau (11) à une deuxième extrémité.

6. Dispositif de forage rotatif suivant l'une quelconque des revendications 1 à 5, dans lequel la tige (27) a un diamètre inférieur à celui de l'aiguille de forage creuse (7).

7. Dispositif de forage rotatif suivant l'une quelconque des revendications 1 à 6, dans lequel la tige (27) a un diamètre externe qui est sensiblement le même qu'un diamètre interne de l'aiguille de forage creuse (7).

8. Dispositif de forage rotatif suivant l'une quelconque des revendications 1 à 7, dans lequel l'adaptateur comprend en outre un moyeu d'adaptateur (29) complémentaire pour être raccordé au moyeu d'aiguille rotatif (13) et pour s'accrocher à celui-ci de sorte que, pendant l'accrochage, la rotation du moyeu d'adaptateur (29) fait tourner le moyeu de l'aiguille rotatif (13).

9. Dispositif de forage rotatif suivant l'une quelconque des revendications 1 à 8, dans lequel le dispositif comprend en outre un chapeau (5) pour être raccordé au moyeu d'aiguille rotatif (13) de sorte que, lorsqu'il est raccordé au moyeu d'aiguille rotatif (13), le chapeau (5) isole et protège la pointe acérée (9) et l'aiguille de forage creuse (7).
